# EUROPEAN PATENT APPLICATION

(11) **EP 3 430 978 A1**
(43) Date of publication of application: **23.01.2019**
(21) Application number: 17181640.8
(22) Date of filing: 17.07.2017
(51) Int. Cl.: A61B 5/00, A61B 5/11, A61B 5/16

(54) **SYSTEM AND METHOD FOR LOGGING SUBJECTIVE AND OBJECTIVE PATIENT DATA AND FOR DETECTING RELEVANT EVENTS**

(71) Applicant: MINDPAX s.r.o., 128 00 Praha 2 (CZ)
(72) Inventor: Novák, Jan, 156 00 Praha 5 (CZ)
(74) Representative: Lahrtz, Fritz

(57) **Abstract**

The invention relates to systems, devices and methods for logging subjective and objective data, in particular data related to mood and schizophrenia disorders. Furthermore, the invention relates to systems, devices and methods for detecting relevant events in the measured data, in particular for detecting mood swings and changes in social rhythmicity. The systems comprise a collection device (1) and a collection station (7), wherein the collection device (1) is connectable to the collection station (7) for data transmission, the collection device (1) comprising a unit (20) for measuring physical activity and a central unit (2) for data processing, wherein the unit (20) for measuring physical activity comprises a digital accelerometer (13) for measuring movement acceleration values, wherein the central unit (2) comprises a signal processor (16) and a memory (24) connected to the signal processor (16), the central unit (2) being adapted for storing the data received from the unit (20) for measuring the physical activity in the memory (24), and for transmitting the stored data to the collection station (7) according to a transmission scheme.

## Description

### TECHNICAL FIELD

The invention relates to systems, devices and methods for logging subjective and objective data, in particular data related to mood and schizophrenia disorders.

Furthermore, the invention relates to systems, devices and methods for detecting relevant events in the logged data, in particular for detecting mood swings and changes in social rhythmicity.

### BACKGROUND

Human life reflects a regular rhythmicity of normal daily activities. It is governed mainly by the sleep-wake cycle and regular physical exercise linked with everyday activities. This cyclic pattern is generally called social rhythmicity. Social context ensures, under normal circumstances, its considerable regularity (getting up, going to work, running a household, going to bed) and, at the same time, quite naturally, also its personalized character.

Change in social rhythms, sleep disruption and proportional change in affective tuning (subjective mood) precedes the repeated outbreaks of bipolar affective disorder (BAD) and schizophrenia. Both disorders represent severe, chronic psychiatric disorders with a significant impairment in the social and professional spheres. In case of a relapse, i.e. a significant deterioration of clinical condition, hospitalization, or a sharp proportional alteration in the subjective mood, of BAD or schizophrenia, often a long-term hospitalization of the patient is necessary. This represents a huge burden for the patient and his/her closest relatives and also results in high costs for the society. Consequently, the detection of changes in social rhythmicity is also important from the medical point of view.

Currently there is a lack of commercial devices capable of automatically detecting and evaluating combined changes in subjective affective tuning and social rhythmicity. Devices for detecting and evaluating combined changes in subjective affective tuning and social rhythmicity have the potential for early detection of impending relapses of severe psychiatric disorders, and early pharmacological intervention.

US 2009/0149778 A1 discloses a depression detection system and method based on an actimetric sensor unit and a computer program to obtain a depression detection result based on the measured activity of the patient. A core idea of the disclosed system is to utilize data representing disturbances of the rest-activity rhythm of a patient, especially disturbances of a patient's sleep, as an indicator of depression. An actimetric technique is used to provide an objective measure for depression or a relapse into depression. The reference, however, does disclose how to perform the continuous detection of changes in affective tuning of the patient. It does not allow continuous data transmision and processing, and does not emit any warning in the case of an increased risk of relapse. Consequently, the disclosed system is more appropriate for data collection for research purposes, as opposed to clinical use.

### DISCLOSURE OF THE INVENTION

According to a first aspect, the present invention relates to a system for logging subjective and objective patient data comprising at least a collection device and a collection station, wherein the collection device is connectable to the collection station for data transmission, the collection device comprising a unit for measuring physical activity and a central unit for data processing, wherein the unit for measuring physical activity comprises a digital accelerometer for measuring movement acceleration values, wherein the central unit comprises a signal processor and a memory connected to the signal processor, the central unit being adapted for storing the data received from the unit for measuring the physical activity in the memory, and for transmitting the stored data to the collection station according to a transmission scheme.

The term "subjective patient data" means data which are assessed and evaluated by the patient him/herself. Subjective data may comprise personal parameters, in particular personal parameters associated with a scale, especially a mood value on a mood scale, a mood change value on a mood change scale, a sleep quality value on a sleep quality scale, a sleep quality change value on a sleep quality change scale, anxiety and energy scale, different questionnaires which are customizable, change of medication and dose initiated by the patient or medical doctor, sleep duration, time to getting to bed, time to get asleep, time to wake up, time to get up, level of energy or tiredness after getting up, as well as life changes like lost of relatives, moving to new address, visit of relatives, travelling across time-zones or night shifts in job..

The term "objective patient data" means data which are measured by a system component, or data derived from data measured by a system component. Objective data may comprise e.g. the movement acceleration values measured by the unit for measuring physical activity. In addition, objective data may comprise data derived from the data on acceleration, as well as additional objective data. These data comprise data on sleep duration, sleep onset and offset, time of getting to and leaving, overall activity, naps during day, as well as data on circadian rhythmicity analysis like acrophase, mesor, period or amplitude and intradaily variability, interdaily stability, the least active five-hour period or the most active ten-hour period.

The patient may generally be any patient wherein the combined measurement of objective and subjective data may be beneficial for diagnostic purposes.

The "collection device" may allow for obtaining and logging at least a part of the subjective and/or objective patient data.

In an embodiment, the collection device is a wristwatch being attachable to the wrist, preferably on the non-dominant hand. Alternatively, the collection device may comprise means for being attached to a belt onto the arm or around the hips. The collection device may comprise an output means such as a visualization unit. collection device may comprise input units, e.g. for controlling the information on the output means. The collection device may comprise a communication unit for transmitting the stored data to the collection station.

The "collection station" allows for the collection of the data obtained by the collection device. In an embodiment, the collection station is a smartphone. In another embodiment, the collection station is a docketing station, e.g. a device permanently attached to an electrical network such as local area network e.g. installed in the apartment of the patient.

The unit for measuring physical activity may comprise the digital accelerometer, an amplifier and a broadband filter. Especially, the digital accelerometer, the amplifier and the broadband filter may be connected in this order. The accelerometer measures the proper acceleration of a body where it is being attached to, i.e. the rate of change of the velocity of the body. The acceleration data may be measured in three orthogonal axes. The movement acceleration values may be amplified, filtered and stored.

The communication between the collection device and the collection station is performed according to a "transmission scheme". The transmission scheme may be a manual transmission scheme or an automatic transmission scheme. Manual data transmission may be triggered by pressing a button. Automatic transmission may be triggered by a time scheme, i.e. after a defined time interval, the data will automatically be sent from the collection device to the collection station. The data transmission from the collection device to the collection station may happen in regular intervals like once an hour, once in three, five, 10 or 20 hours, once a day, once every two, three, four or five days. The data transmission may use any suitable protocol, e.g. GPRS, 3G or 4G. The transmission of the stored data, e.g. once the defined time interval is over, may also depend on whether the docketing station has been detected by the collection station.

According to an embodiment, the system comprises an input interface for collecting a personal parameter associated with a scale. The data received from the input interface for collecting the personal parameter is transmitted to the collection station. The personal parameter can be entered e.g. via a smartphone, a wristwatch or any other suitable means. The personal parameter may be characterized by the patient by entering a value on a scale ranging from -5 passing by 0 to 5. The value range is exemplarily and may also be from -10 to 10, from -3 to 3, from -2 to 2, or may only comprise positive values such as from 0 to 10 or from 0 to 5. The scale may also be associated with a development and comprise a value for "lower", another value for "equal" and another value for "higher".

According to an embodiment, the collection device includes the input interface for collecting the personal parameter. In this embodiment, the central unit is also adapted for storing the data received from the input interface for collecting the personal parameter in the memory. The system therefore includes a collection device which is designed for the integrated collection of subjective data and objective data, such as data related to the patient's physical activity, in particular the patient's movement data. Subsequently, the collection device mediates the transmission and the processing of the combined data.

The input interface for collecting the personal parameter may comprise a start means for recording an event, navigation means for the selection of a parameter value from the parameter scale, and an OK means for the confirmation of a selected parameter value. The "means" may have any suitable form, and they may be in particular in the form of mechanical or computer buttons or touch sensitive panels. If, according to a preferred embodiment as also discussed in more detail below, the parameter is "mood", then the start means may be labelled as "Mood". An alternative configuration of the input interface may comprise only one means, e.g. one "parameter button", wherein the pressing time of the means in combination with a display showing the information of the parameter to be entered may be used for fulfilling the functions "start", "navigation in the scale" and "confirmation".

According to another embodiment, the input interface for collecting the personal parameter values does not exist in the collection device. The personal parameter may then be recorded by using an application installed on a mobile phone or smartphone. Also in this case, the collection device may comprise an input means with a number of functions on it. In particular, there may be one means, e.g. one button, wherein the short pressing of the button may lead to a display showing a) status information regarding the condition of the patient, b) a watch function and c) fitness information, e.g. number of steps in a loop, and the long pressing of the button may lead to the initiation of data transfer or the like.

The system may comprise a personal parameter input prompting alarm system. By this means the patient may be driven to regularly enter his/her personal parameter, e.g., every day or every week, as a response to a discrete acoustic signal and an alarm on the display of the collection device. Entering the data preferably takes only few seconds.

In an embodiment, the collection device comprises a communication unit for providing the transmission of the saved data to the collection station. The communication unit may comprise a cable interface or a wireless transmission interface, in particular a USB, Bluetooth, WLAN, infrared or radio interface.

The collection device, in particular the communication unit, may comprise a transfer button or a module for detecting a gesture for triggering the transmission of the stored data. The transfer button triggers the transmission of the stored data upon pressing. The module for detecting a gesture triggers the transmission of the stored data upon detection of the presence of the gesture.

The communication unit may comprise a module for detecting the presence of the collection station for the automatic data transmission. The communication unit may also comprise timers for triggering the automatic data transmission.

In particular, the collection station may be a docketing station and the collection device, in particular the communication unit, may comprise a means for detecting the presence of the docketing station and for automatically transmitting the data every time the collection device detects the docketing station. Subjective and objective patient data, e.g. physical activity data and daily records of the personal parameter, are then automatically transmitted from the collection device the patient wears on his/her wrist as a regular watch into the collection station and that is done every time the two devices get close. Advantageously, data collection is independent from the patient, in particular from the patient's current mood.

According to another aspect, a system for detecting relevant events in collected subjective and objective patient data is provided, the system comprising the system for logging subjective and objective patient data as described above and a central station connected to the collection station, the central station comprising an analyzer tool adapted for detecting the relevant events in the data received from the collection station.

The analyzer is equipped with and may be performing a method of data analysis, e.g. by means of artificial intelligence. At the central station, the automatic calculations of predictions are performed. Based on a computer implemented method, the system is capable of mathematically analyzing the ongoing changes in the integrated data and to optimize the predictive algorithm.

The central station may be a remote server, e.g. a cloud server. The central station may also serve as a long-term storage of combined data obtained from a plurality of patients. The system also mediates the continuous analysis of pooled data.

The collection station may be a cable-bound or wireless transfer element between the collection device and the central station. The data transmission from the collection station to the central station may happen on a periodic basis, related or unrelated to the data transmission scheme of the collection device and the central station. Data transmission may also be implemented on a permanent basis.

The system for detecting relevant events in collected subjective and objective patient data may comprise means for providing alarms upon the detection of relevant events. In particular, the collection device may comprise an output means and the central station may comprise an alarm generator, wherein the collection device is adapted for signaling the alarms received from the central station through the output means. The output means may comprise a display unit comprising an alarm generator adjusted for generating vibration and/or acoustic alarm using an audio-vibration unit. By means of an information feedback loop, the system may thus communicate a relevant event to the patient. This can be done by sending an automated e-mail alarm warning or any other communication alarm such as text message. The alarm may include a call for therapeutic intervention as discussed below. Alarms as the result of the prediction may also be sent by e-mail or any other communication means to the patient's attending physician or any other relevant person.

According to another aspect, collection devices and collection stations are provided, the collection devices and collection stations being prepared for being used in one of the systems for logging subjective and objective patient data as described above.

According to another aspect, a central station is provided, the central station being prepared for being used in one of the systems for detecting relevant events in collected subjective and objective patient data as described above.

According to yet another aspect, methods for logging subjective and objective patient data and methods for detecting relevant events in collected subjective and objective patient data are provided. Preferably, the systems and devices as described herein are configured for performing the methods as described herein. Therefore, features which are described in the context of the systems and devices are understood to be disclosed for the methods, and, vice versa, features which are described in the context of the methods are understood to be disclosed for the systems and devices as well.

In another aspect, the invention relates to a method for logging subjective and objective patient data comprising the steps of:
measuring, by a digital accelerometer of a unit for measuring physical activity, movement acceleration values of a patient,
storing the data from the digital accelerometer as movement data in a memory of a central unit and
transmitting the movement data from the memory of the central unit to a collection station according to a transmission scheme.

The method may further comprise the step of storing data from an input interface for collecting a personal parameter in the collection station, in particular manually entered data.

According to an embodiment, the movement acceleration values are measured with a non-uniform sensor resolution over the measurement range. In other terms, different magnitude ranges of the movement acceleration are measured with different precisions.

The term "sensor resolution" of the unit for measuring physical activity refers to the measurement resolution. The sensor resolution may be understood as the smallest change the unit for measuring physical activity can detect.

The term "non-uniform" means that the sensor resolution is non-constant over the measurement range. In particular, the resolution can be asymmetric, such as e.g. exponential or polynomial (in steps).

Specifically, the sensor resolution may be such that acceleration values of lower magnitudes are measured with a higher sensor resolution than values of higher magnitudes.

As an example, an acceleration value range from 0 to 4 g may be divided into 256 subranges, wherein the first 128 subranges are attributed to the value range from 0 to 1 g, the next 64 subranges are attributed to the value range from 1 to 2 g and the final subranges are attributed to the value range from 2 to 4 g. However, the acceleration value range may be adapted to the purpose and may also be e.g. from 0 to 3 g, from 0 to 2 g or from 0 to 1 g. Also the number of the subranges can be adapted to the purpose and may range e.g. from 32 to 1024. Furthermore, the acceleration value range may be split up into two, three (as in the example above), four, five or more value ranges, also depending on the purpose.

The movement data measured by the accelerometer may be further preprocessed by the unit for measuring physical activity and/or by the central unit. In an embodiment, an acceleration value with a largest magnitude or an average value of all measured acceleration values is selected and stored for each data aggregation time interval.

The "data aggregation time interval" may be a fixed interval in the range from 0.01 s to 10 s, in particular from 0.1 s to 1 s, in particular from 0.1 s to 0.2 s.

According to another aspect, the present invention relates to a method for detecting relevant events in subjective and objective patient data comprising the steps of:
performing the method for logging subjective and objective patient data as described above,
transmitting the logged data to a central station and
calculating relevant events from the movement data and, if applicable, from the data relating to the personal parameter by comparing the logged data to reference standard data.

Aspects of the disclosed embodiments may be implemented in the general context of computer-executable instructions, such as program modules, being executed by a computer, computer server, or device containing a processor. Generally, program modules or protocols include routines, programs, objects, components, data structures, hardware executable instructions may perform particular tasks or implement particular abstract data handling. Aspects of the disclosed embodiments may also be practiced in distributed computing environments where tasks are performed by remote processing devices (processors, microprocessors, computing systems, FPGAs, programmable ICs, etc.) that are linked through a communications network, such cloud servers over the internet. In a distributed computing environment, program modules and hardware executable instructions may be located in both local and remote storage media such as memory storage devices (including non-transitory storage media). Those skilled in the art will appreciate that, given the description of the modules comprising the disclosed embodiments provided in this specification, it is a routine matter to provide working systems which will work on a variety of known and commonly available technologies capable of incorporating the features described herein. Additionally, the methods described herein can be implemented in a hardware-readable storage medium (including non-transitory computer- readable media) with an executable program stored thereon, wherein said executable program instructs the processing hardware perform the method steps.

Therefore, according to another aspect, computer programs are provided which cause processors of the systems or devices disclosed above to perform one of the methods disclosed above when executed.

Software modules of the central unit, pre-processing means, amplifiers and filters are *functional* units which are not necessarily physically separated from each other. Several functions may be realized in the form of one single physical unit, for instance implemented in software. Furthermore, amplifiers and filters but also the pre-processing means may equally be realized as hardware, for example as ASIC (Application Specific Integrated Circuit), microcontrollers, or the like.

In case a medical intervention is needed, the physician may, after he/she may have discussed the possible consequences with the patient, take the appropriate measures depending on the disease. This may include the administration or the adjustment of the medication needed for treating the patient's disease, other measures like psychotherapy, light therapy, rTMS (Repetitive Transcranial Magnetic Stimulation) or physical therapy as well as surgery.

In an embodiment, the subjective and objective patient data are related to mood and schizophrenia disorders. Mood and schizophrenia disorders according to the invention may be depression, BAD, schizophrenia, as well as bipolar, depression and schizophrenia disorders.

During the period before the relapse of BAD and schizophrenia, there are changes in the patient's mood, regularity of his/her daily regimen and the nature of sleep. These changes concern the shortening and fragmentation of sleep, changes in the time when the patient goes to bed and gets up, changes in the regularity of daily activities, and other variables. The detection of changes in the regularity of social rhythms, therefore, enables the prediction of the onset of another phase of the disease. The system of the present invention is especially suited for said detection and said prediction.

In an embodiment, a system for logging subjective and objective patient data related to mood and schizophrenia disorders is provided that comprises at least a collection device and a collection station, wherein the collection device is connectable to the collection station for data transmission, the collection device comprising a unit for measuring physical activity and a central unit for data processing, the unit for measuring physical activity comprising a digital accelerometer for measuring movement acceleration values, the central unit comprising a signal processor and a memory connected to the signal processor, wherein the central unit is adapted for storing the data received from the unit for measuring the physical activity in the memory, and for transmitting the stored data to the collection station according to a transmission scheme.

The following data may be considered and collected as subjective patient data related to mood and schizophrenia disorders: a mood value on a mood scale, a mood change value on a mood change scale, a sleep quality value on a sleep quality scale, a sleep quality change value on a sleep quality change scale, anxiety and energy scale, different questionnaires which are customizable, change of medication and dose initiated by the patient or medical doctor, sleep duration, time to getting to bed, time to get asleep, time to wake up, time to get up, level of energy or tiredness after getting up, as well as life changes like lost of relatives, moving to new address, visit of relatives, travelling across time-zones or night shifts in job.

Movement acceleration values, especially movement acceleration values from a range of 0 to 2 g, may be considered and collected as objective patient data that are related to mood and schizophrenia disorders. In addition, also body temperature, pulse, blood pressure, or data on skin galvanic response may be collected. Furthermore, also data obtained by electroencephalography may be collected.

According to an embodiment, system comprises an input interface for collecting mood values associated with a mood scale. The mood scale may e.g. range from - 5 to 5, wherein -5 may correspond to the deepest depression, 0 may stand for stable mood and 5 may correspond to the greatest mania. The mood scale may be also range from -10 to 10, from -3 to 3, from -2 to 2, or may only comprise positive values such as from 0 to 10 or from 0 to 5. Alternatively, the input interface may collect mood change values associated with mood change scale, e.g. comprising a value for "lower", another value for "equal" and another value for "higher" such that the patient may specify the mood changes from day to day, or the like.

According to an embodiment, the collection device is designed for the integrated collection of data on current affective tuning, i.e. the current mood score, and the patient's movement data.

In an embodiment, a system for detecting mood swings and changes in social rhythmicity is provided, the system comprising the system for logging subjective and objective patient data related to mood and schizophrenia disorders as described above and a central station connected to the collection station, the central station comprising an analyzer tool adapted for detecting the mood swings and changes in social rhythmicity using the data received from the collection station.

The term "mood swings" may mean an extreme or rapid change in mood.

The term "changes in social rhythmicity" may mean a rapid change in social activities throughout the week.

The system may provide alarms upon the detection of relevant events. The alarms may warn the patient and/or the patient's attending physician and include a call for therapeutic intervention. In case of mood disorders, this therapeutic intervention may aim at the prevention of an relapse and may include the administration of antipsychotics and mood stabilizers e.g. based on lithium/valproate like lurasidone, olanzapine and quetiapine or the beginning of a psychotherapy, light or rTMS therapy.

Consequently, in another aspect of the invention, the invention also relates to an antipsychotic drug for use in a method for treating a mood disorder or for preventing a relapse in the context of a mood or schizophrenic disorder, the method comprises performing the method of the invention and the administration of the antipsychotic drug. The antipsychotic drug may be administered in an amount sufficient to treat the mood disorder or to prevent the relapse.

Likewise, the invention also relates to a method for treating a mood disorder or preventing a relapse in the context of a mood disorder, the method including performing the method of the invention and administering the antipsychotic drug in an amount sufficient to treat the mood disorder or to prevent the relapse.

According to an embodiment, a method for logging subjective and objective patient data related to mood and schizophrenia disorders comprises the steps of: measuring, by the digital accelerometer of the unit for measuring physical activity, the movement acceleration values of a patient,
storing the data from the digital accelerometer as movement data in the memory of the central unit and
transmitting the movement data from of the memory of the central unit to the collection station according to a transmission scheme.

The method provides the collection of data regarding regularity in the social rhythmicity of an individual, e.g. data on structure and regularity of daily activities,

If movement acceleration values are measured with a non-uniform sensor resolution over the measurement range, in particular where low g values are measured more precisely than high g values, one advantage is that it enables drawing conclusions about sleep quality where the low g values are expected to predominate. Advantageously, any subtitle movements during sleep as small turns in bed can be detected. Therefore, the system may thus enable the automated collection of data regarding the nature and the quality of sleep.

Additionally or alternatively, the monitoring of sleep may be performed by using a Gₘₐₓ aggregation method meaning that in a certain period of time a maximum value of the movement acceleration values of the patient is selected and regularly stored. As to the memory management, the periodicity of the selection and storage of the maximum value of the movement acceleration values may be between 10 s and 2 min, especially between 20 s and 40 s, in order to save battery (saving values in memory is time consuming) and memory.

As to the display management, the collection device display may be kept off unless a button is pressed twice, hence prolonging battery life.

As to the data transfer management, the collection device may scan the collection station several times per day only, especially once in a fixed interval of 6 hours to 12 hours, e.g. each 8 hours, hence prolonging battery life.

According to an embodiment, a method for detecting mood swings and changes in social rhythmicity comprises the steps of:
performing the method for logging subjective and objective patient data related to mood and schizophrenia disorders as described above,
transmitting the logged data to a central station and
identifying mood swings and changes in social rhythmicity by comparing the logged data to reference standard data.

According to an embodiment, after transmission of the movement and mood data from the memory of the central unit to the collection station and from the collection station to the analyzer tool of the central station, the data is compared to reference data in terms of a reference standard of previous BAP or schizophrenia relapse saved in the central station. Under the term "reference standard" the characteristic course of a set of predictive parameters, or the characteristic course of an algorithmic or mathematical combination of predictive parameters before the relevant event happens/happened is understood.

Specifically, the analysis may involve a comparison between a current data window and one or more reference standard windows, wherein the term "window" denotes a defined time period. If a current data window is constituted by the current physical data obtained during the last N weeks of measurement, the reference "window" will contain also N weeks of reference measurements.

The "reference standard" may be taken from the same patient and is, e.g., obtained from the patient's attending physician. In the case when a new patient enters the study, there is typically no individualized reference data available, e.g. no reference standard of relapse and hospitalization for this patient. Then, general data, e.g. a reference standard of BAD or schizophrenia relapse which has been obtained from other patients, e.g. patients who have participated in a pilot study, may be used as the reference standard.

If the sequences in the current data window are similar to one of the previously obtained reference standards windows, the alarm may be declared. Mutual similarity of the current data window and the reference standard window may be calculated e.g. using the method of dynamic warping (DTW) and hidden Markov Models (HMM) or neuronal networks (NN).

In an embodiment, thus, the recording of individual changes in social rhythms before the relapse happens, serves as a basis for future early prediction of the imminent phase of the disorder.

### BRIEF DESCRIPTION OF THE FIGURES

Disclosed embodiments of the invention are illustrated by way of example, and not by way of limitation, in the figures of the accompanying drawings.
FIG. 1 shows a schematic illustration of the architecture of the system for detection of mood swing and changes in social rhythmicity according to an embodiment.
FIG. 2 shows a simplified schematic illustration of a collection device according to an embodiment.
FIG. 3 shows a more detailed block diagram of the collection device according to an embodiment.
FIG. 4 shows a simplified schematic illustration of the collection station according to an embodiment.
FIG. 5 shows a flow chart of a method for BAD / schizophrenia relapse prediction according to an embodiment.
FIG. 6 shows a flow chart for updating the relapse reference standard according to an embodiment.
FIG. 7 shows a schematic representation of the calculation of the window.

### DETAILED DESCRIPTION OF THE FIGURES

Although being described in relation to the detection of mood swings and changes in social rhythmicity, the systems and methods mentioned in the following refer to data collection and to detecting relevant events in collected data in general.

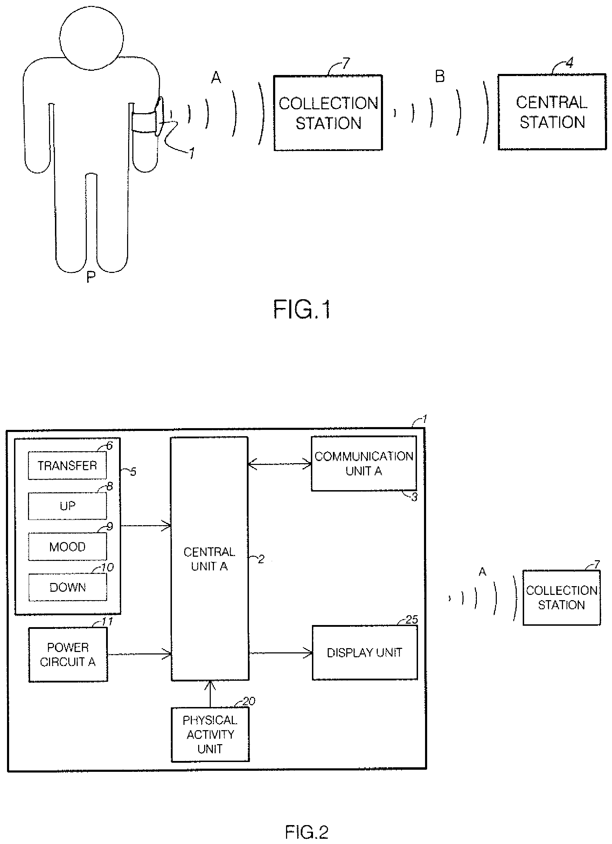

FIG. 1 illustrates the architecture of a system for detecting mood swings and changes in social rhythmicity according to a particular embodiment.

In this embodiment, a patient P's physical activity and mood values are transmitted from a collection device 1 to a collection station 7 by means of wireless transfer A. The data is transmitted from the collection station 7 to a central station 4 by wireless transfer B.

In an alternative embodiment, the patient's mood values are inputted into a mobile phone, smartphone, tablet computer, PC or the like (not depicted) and either transmitted to the collection station 7 or directly to the central station 4. The mobile phone, smartphone, tablet computer, PC or the like is equipped with appropriate technical specifications and with a suitable application for this purpose.

As shown in the schematic simplified illustration in FIG. 2, according to an embodiment, the collection device 1 includes a unit 20 for measuring physical activity, a central unit 2 for data processing, an input interface 5 for the manual entry of data, a communication unit 3 for data transfer and an output means 25 for displaying data.

The unit 20 for measuring physical activity measures the physical activity in the three planes X, Y, Z by using a digital accelerometer 13 containing an actigraphic sensor.

The central unit 2 pre-processes the movement data and then the data processed by this means are transmitted via the communication unit 3 to the collection station 7. The communication unit 3 may involve a GSM/GPRS/3G/4G modem and/or hardware ports such as a USB port.

The input interface 5 is used for entering the patient's current mood and for the initiation of data transfer. The input interface 5 comprises a transfer button 6, a mood button 9, and navigation buttons 9 and 10. Exemplarily depicted are an up button 9 and a down button 10.

The transfer of data to the collection station 7 is performed after a transfer button 6 has been pressed. In an alternative embodiment (not depicted), the data is transmitted from the device 1 to the collection station 7 automatically, e.g. every time the collection device 1 detects the collection station 7.

In an exemplary embodiment, after the mood button 9 has been pressed, a flashing number 0 is displayed on the output means 25; by pressing the up button 8, the number is increased by one unit, by pressing the down button 10, the number is decreased by one unit. By pressing the mood button 9 again or an extra button such as an OK button (not depicted), the number is saved in the memory. By this means, the patient may enter the value of his/her current mood in the range of e.g. -5 to +5.

The energy source for the collection device 1 is a battery constituting a part of the power circuit 11. The battery may be a rechargeable battery. In an preferred embodiment, the battery is a long-life battery contributing to a better power management.

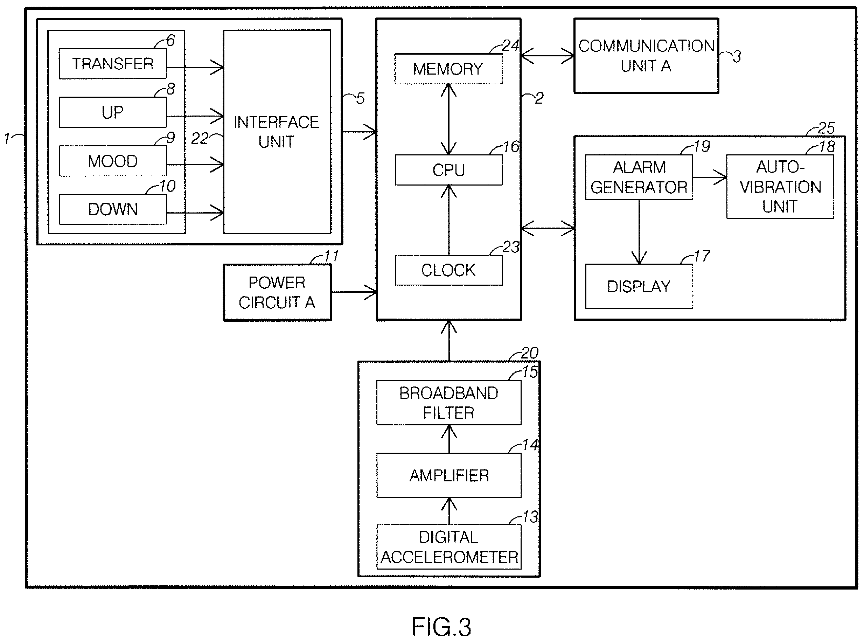

FIG. 3 shows a more detailed block diagram of the collection device 1 according to an embodiment of the invention.

The mood button 9, up button 8, down button 10, and transfer button 6 are connected to the central unit 2 via an interface unit 22. After the mood value has been confirmed, date and time is displayed on the display 17 of the output means 25.

The display 17 may further show time, date, overall physical activity and a list of alarms. The display unit 25 is linked to the central unit 2 by a data bus.

The unit 20 for measuring physical activity comprises a digital accelerometer 13, an amplifier 14 and a broadband filter 15. The digital accelerometer 13 measures the physical activity of movement and its output is an accelerometer signal. The digital accelerometer 13 is connected via the amplifier 14 and the broadband filter 15 to the processor 16 of the central unit 2, represented in Figure 3 as CPU. Physical activity and manually generated mood (with the mood button 9) represent the input for the central unit 2.

The central unit 2 is also bi-directionally connected to the communication unit 3 and to the memory 24. Furthermore, the central unit 2 comprises a reference clock 23 which generates the time for the signal processor 16.

Furthermore, the central unit 2 is connected to an alarm generator 19. The alarm generator 19 may output alarms through the display 17 and, by way of example, through an audio-vibration unit 18. Alarm signals may be received from the central station 4 by the communication unit 3 (the communication channel is not depicted).

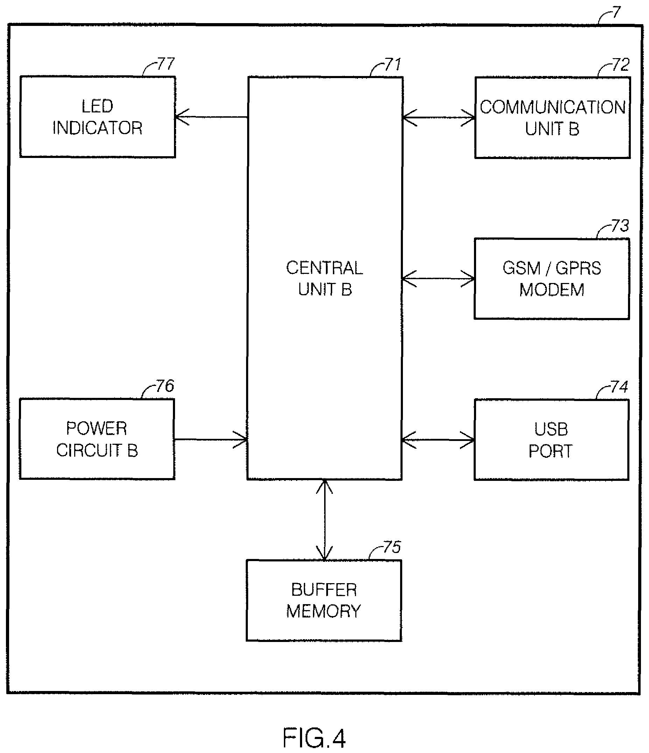

FIG. 4 shows an embodiment of a collection station 7 that uses a second central unit 71, a second communication unit 72, a GSM/GPRS/3G/4G modem 73, a USB interface 74, a buffer memory 75, a power circuit 76, and LED indicators 77.

Data from the collection device 1 is received by the second communication unit 72 and saved in the buffer memory 75. After the completion of data reception, the data is subsequently transmitted to the central station 4. The transmission of data to and/or from the collection station 7 may be visualized by the LED indicators 77. The second central unit 71 controls the transfer to and/or from the collection station 7 and the LED indicator 77 visualization. The power circuit 76 is a source of energy for the collection station 7. Data to and/or from the collection station 7 may be wirelessly transmitted via the GSM/GPRS/3G/4G modem 73 and/or via the USB port 74.

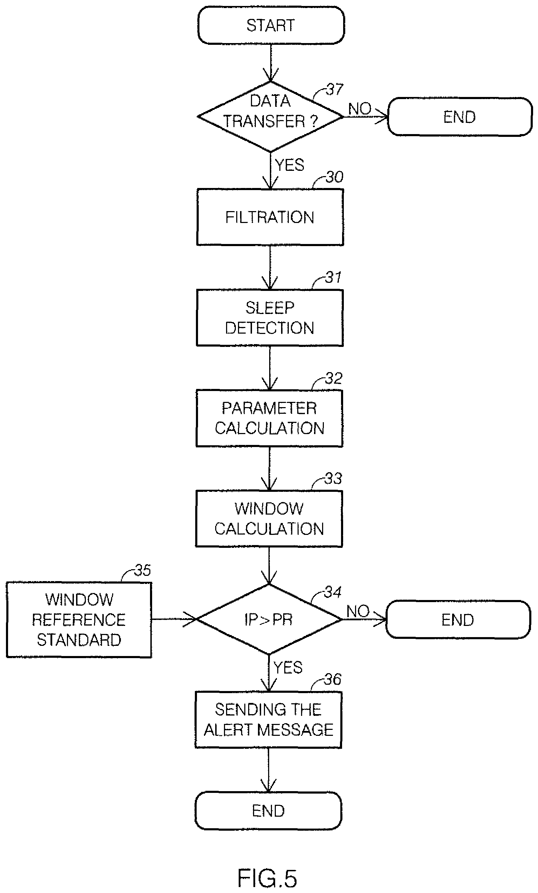

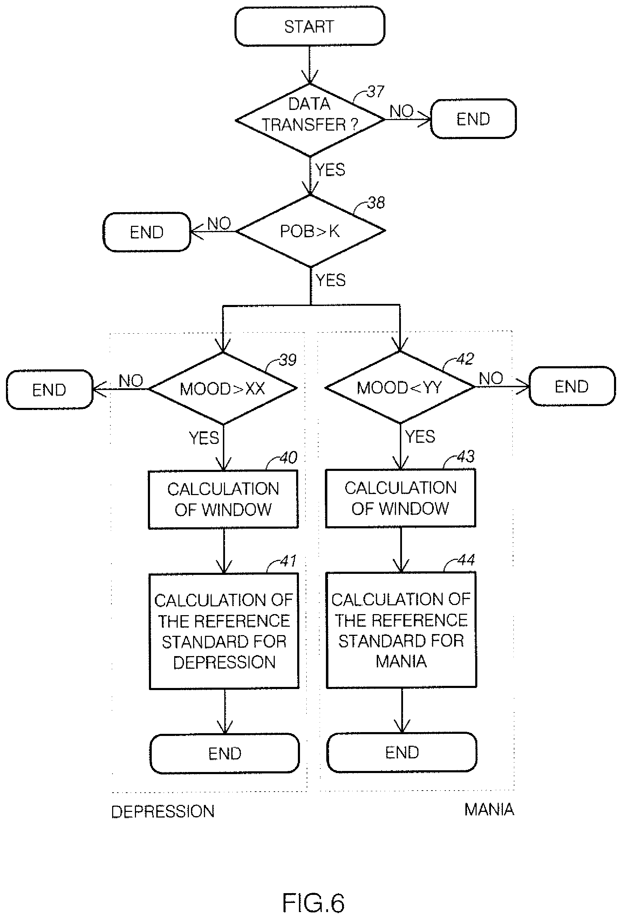

According to an embodiment, after each data transmission operation, the prediction of relapse is assessed (see FIG. 5) and the reference standard of BAD or schizophrenia relapse for the individual patient is updated (see FIG. 6).

FIG. 5 illustrates a flowchart of a particular embodiment for detection of bipolar disorder/schizophrenia relapse.

In this particular embodiment, and without limitation, a first step 37 first involves the transmission of data. Data transmission is carried out e.g. by means of an automatic wireless connection of the collection device 1 to the collection station 7 in the case that the collection station 7 is within reach of the collection device 1. Then, the data is sent from the collection station 7 to the central station 4, which may also happen by means of a wireless connection.

A second step 30 involves filtration using a median filter. Any filtering and pre-processing of data may also be performed prior to data transmission, e.g. on the collection device 1.

In a third step 31 sleep and daily activity is detected in the signal of physical activity.

In a fourth step 32, the calculation of parameters, such as the duration of sleep, sleep fragmentation, or number of segments classified as sleep, is performed in the domain of sleep. In the domain of daily activities, segments of rest are also detected. In the domain of circadian rhythmicity, a set of parametric parameters, such as acrophase, mesor, period or amplitude and a set of non-parametric parameters, such as intradaily variability, interdaily stability, the least active five-hour period or the most active ten-hour period are calculated.

In a fifth step 33, a window of duration of N weeks is composed from these parameters. From this data, in a sixth step 34, a current data window is calculated.

The current data window is then compared in the seventh step 34 to one or more reference standard windows 35. The reference standard windows 35 may be related to BAD or schizophrenia relapse.

The result of this comparison is the similarity index IP, which may range from 0, i.e. no similarity, to 1, i.e. if the courses of parameters in the window are identical.

The similarity index IP is calculated using the method of dynamic time warping (DTW) and/or Hidden Markov Models (HMM) and/or neural networks (NN). If the IP similarity index calculated in the seventh step 34 exceeds a threshold Pr, then, in an eighth step 36, an alarm is declared. The alarm is transmitted to the collection device 1 and sent via e-mail or text message to the attending physician.

The update mechanism is shown in FIG. 6.

Let patient P use the collection device 1 for the actual time POB. If the patient P is in the system for more than a minimum period of K weeks, wherein K may be any integer, e.g. K = 12, then the reference standard is updated. The comparison of POB and K is performed a ninth step 38.

If during the use of the system the mood significantly changes to the extreme, then the current data window is added to the window of relapse.

In more detail, if significant mood deterioration is detected in a thirteenth step 39 then, in a fourteenth step 40, the depression reference standard window is called and, subsequently, the current data window is added to and stored as the mania reference standard window in a fifteenth step 41.

An analogous procedure applies in the case of mania relapse: if significant mood improvement is detected in a sixteenth step 42 then, in a seventeenth step 43, the mania reference standard window is called and, subsequently, the current data window is added to and stored as the mania reference standard window in an eighteenth step 44.

An analog method may be performed in the case of schizophrenia. In this case a PSYCHO parameter may be an aggregation from questionnaires used in clinical trials. If during the use of the system the mood significantly changes to the extreme, then the schizophrenia reference standard window is called and, subsequently, the current data window is added to and stored as the schizophrenia reference standard window, leading to an update of the reference standard.

The reference standards may be saved within the parameters of the models (as e.g DTW, HMM or NN).

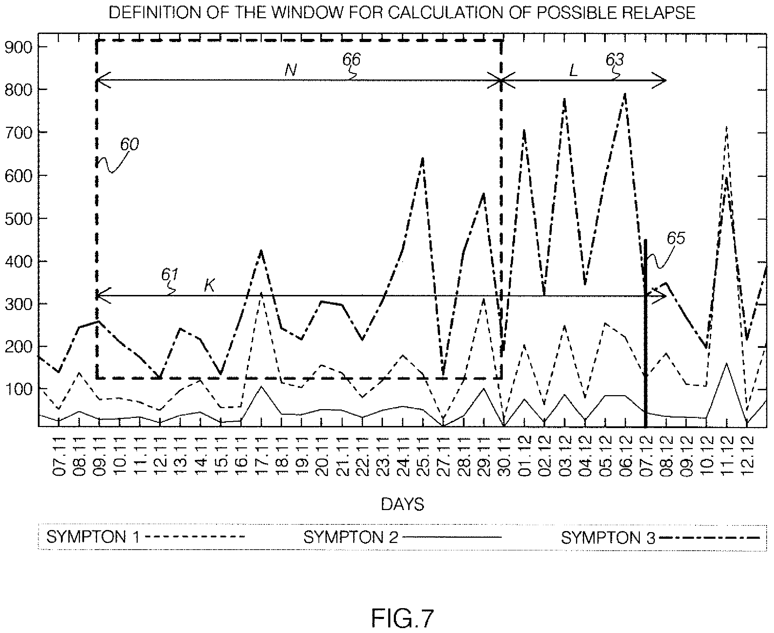

According to an embodiment, the "window" 60 is a period of time before the depression/mania or schizophrenia relapse develop and it is shown in more detail in FIG. 7.

During a certain period of time the patient does not experience any relapse of depression/mania or schizophrenia. The window 60 is a time period that begins K weeks 61 prior to the depressive/manic episode or schizophrenia relapse 65 and ends L week/weeks prior to the depressive/manic episode or schizophrenia relapse 65. Thus, the duration of the window 60, expressed by parameter N 66, equals to the difference of K-L.

Based on the transmitted data, the parameters of the models (e.g., DTW, HMM or NN) for BAD relapse or schizophrenia relapse are re-set using the process of learning in the fifteenth step 41 and in the eighteenth step 44.

The new reference standards for depression/mania or schizophrenia relapse apply only to the given patient P. This process may thus be regarded as personalization and updating of the relapse prediction algorithm according to the current course of the disease observed in the given patient P. After each update, the parameters of models (e.g. HMM, DTW or NN) are modified and stored in the memory (not depicted) of the central station 7.

According to an embodiment, the method of relapse prediction is carried out as a computer program performed in the central station 7. For updating the reference standard, the same or another computer program running in the central station 7 may be used.

It is noted that the disclosed embodiments and examples have been provided merely for the purpose of explanation and are in no way to be construed as limiting. Further, although the systems and methods have been described herein with reference to particular means, materials, steps and embodiments, the actual embodiments are not intended to be limited to the particulars disclosed herein; rather, the system and methods extend to all functionally equivalent structures, methods and uses, such as are within the scope of the appended claims. While particular embodiments have been described, it is understood that, after learning the teachings contained in this disclosure, modifications and generalizations will be apparent to those skilled in the art without departing from the scope of the claims. E. g., those of ordinary skill in the relevant art will understand that they can practice other embodiments without one or more of the details described in the present disclosure.

Furthermore, certain well-known details often associated with medical devices, computing and software technology have not been set forth in the present disclosure to avoid unnecessarily obscuring of the various disclosed embodiments.

## Claims

1. A system for logging subjective and objective patient data related to mood and schizophrenia disorders, the system comprising at least a collection device (1) and a collection station (7), wherein the collection device (1) is connectable to the collection station (7) for data transmission, the collection device (1) comprising a unit (20) for measuring physical activity and a central unit (2) for data processing, wherein the unit (20) for measuring physical activity comprises a digital accelerometer (13) for measuring movement acceleration values, wherein the central unit (2) comprises a signal processor (16) and a memory (24) connected to the signal processor (16), the central unit (2) being adapted for storing the data received from the unit (20) for measuring the physical activity in the memory (24), and for transmitting the stored data to the collection station (7) according to a transmission scheme.

2. The system of claim 1, wherein the collection device (1) is a wristwatch, or comprises means for being attached to a belt.

3. The system of any one of the preceding claims, further comprising an input interface (5) for collecting mood values associated with a mood scale or mood change values associated with a mood change scale.

4. The system of claim 3, wherein the collection device (1) includes the input interface (5) for collecting mood values and wherein the central unit (2) is adapted for storing the data received from the input interface (5) for collecting mood or mood change values in the memory (24).

5. The system of any one of the preceding claims, wherein the collection device (1) comprises a transfer button (6) or a module for detecting a gesture for triggering the transmission of the stored data.

6. The system any of the preceding claims, wherein the collection station (7) is a docketing station, and wherein the collection device (1) comprises a means for detecting the presence of the docketing station and for automatically transmitting the data every time the collection device (1) detects the docketing station.

7. A system for detecting mood swings and changes in social rhythmicity, the system comprising the system for logging subjective and objective patient data related to mood and schizophrenia disorders of any one of the preceding claims and a central station (4) connected to the collection station (7), the central station (4) comprising an analyzer tool for detecting the mood swings and changes in social rhythmicity using the data from the collection station (7).

8. The system of claim 7, wherein the collection device (1) comprises an output means and wherein the central station (4) comprises an alarm generator, wherein the collection device (1) is adapted for signaling the alarms received from the central station (4) through the output means.

9. A collection device (1) prepared for being used in a system for logging subjective and objective patient data related to mood and schizophrenia disorders according to any one of claims 1 to 6.

10. A collection station (7) prepared for being used in a system for logging subjective and objective patient data related to mood and schizophrenia disorders according to any one of claims 1 to 6.

11. A central station (4) prepared for being used in a system for detecting mood swings and changes in social rhythmicity according to any one of claims 7 or 8.

12. A method for logging subjective and objective patient data related to mood and schizophrenia disorders using the system of any one of claims 1 to 6, the method comprising the steps of:
measuring, by the digital accelerometer (13) of the unit (20) for measuring physical activity, the movement acceleration values,
storing the data from the digital accelerometer (13) as movement data in the memory (24) of the central unit (2) and
transmitting the movement data from of the memory (24) of the central unit (2) to the collection station (7) according to a transmission scheme.

13. The method of claim 12, wherein the movement acceleration values are measured with a non-uniform sensor resolution over the measurement range.

14. The method of claim 12 or 13, further comprising the step of:
pre-processing, by the central unit (2), the movement data received from the unit (20) for measuring physical activity, the pre-processing comprising selecting and storing an acceleration value with a largest magnitude for each data aggregation time interval.

15. A method for detecting mood swings and changes in social rhythmicity using the system of any one of claims 7 or 8, the method comprising the steps of:
performing the method for logging subjective and objective patient data related to mood and schizophrenia disorders of any one of claims 12 to 14, transmitting the logged data to a central station and
identifying mood swings and changes in social rhythmicity by comparing the logged data to reference standard data.
